# EUROPÄISCHE PATENTSCHRIFT

(11) **EP 2 372 330 B1**
(45) Veröffentlichungstag und Bekanntmachung des Hinweises auf die Patenterteilung: **16.01.2013**
(21) Anmeldenummer: 10003530.2
(22) Anmeldetag: 31.03.2010
(51) Int. Cl.: G01M 3/06, G01N 29/032, A61M 1/36

(54) **Luftblasensensor**
Air bubble sensor
Détecteur de bulles

(43) Veröffentlichungstag der Anmeldung: 05.10.2011
(73) Patentinhaber: LIFEBRIDGE Medizintechnik AG, 84539 Ampfing (DE)
(72) Erfinder: Sagebiel, Florian, 84562 Mettenheim (DE)
(74) Vertreter: Manitz, Finsterwald & Partner GbR

(56) Entgegenhaltungen:
- EP-A1- 0 778 465
- US-A- 4 237 720

## Beschreibung

Die vorliegende Erfindung betrifft einen Luftblasensensor nach dem Oberbegriff des Anspruchs 1 mit einer Halterung, an der zumindest ein Ultraschallsensor angeordnet ist, um Luft- und/oder Gasblasen in einer strömenden Flüssigkeit zu detektieren (vgl. US-A-4 237 720).

Derartige Luftblasensensoren sind auch aus der Praxis bekannt und dienen beispielsweise bei mobilen Herz-Lungen-Maschinen zur Erhöhung der Sicherheit des Patienten gegen Luftembolie. Sobald durch einen solchen Luftblasensensor Luft in einem blutführenden Schlauch detektiert wird, können Sicherheitsklemmen aktiviert werden, die ein Weiterleiten von Luftblasen in den Körper des Patienten verhindern.

Bei bekannten Luftblasensensoren wird ein blutführender Schlauch in die Halterung eingelegt, was bei einem Notfalleinsatz wertvolle Zeit kosten kann. Weiterhin sind Luftblasensensoren bekannt, bei denen vor Einlegen des Schlauches in die Halterung ein Koppelmedium eingebracht werden muss, um die Ankopplung zwischen Luftblasensensor und Schlauch zu verbessern. Dies ist insbesondere bei Notfalleinsätzen ebenfalls zeitaufwändig und fehleranällig.

Es ist die Aufgabe der vorliegenden Erfindung, einen Luftblasensensor der eingangs genannten Art im Hinblick auf eine Verwendung bei Notfalleinsätzen zu optimieren. Die Lösung dieser Aufgabe erfolgt durch die Merkmale des Anspruchs 1. Der Luftblasensensor kann mit Hilfe der Anschlussstücke lange vor einem Einsatz an (dann) blutführende Schläuche angeschlossen werden, sodass bei Inbetriebnahme des Luftblasensensors ein Einlegen eines Schlauches oder gar ein Vorsehen eines Koppelmediums nicht erforderlich ist. Durch Integration des Ultraschallsensors und des Strömungskanals sowie der Anschlussstücke in die Halterung ist ein einziges Bauteil geschaffen, das insbesondere als Einwegteil ausgebildet sein kann und das nach einem Einsatz entsorgt werden kann. Der Luftblasensensor gemäß der Erfindung kann wirtschaftlich hergestellt werden und bereits in der Produktion, beispielsweise an einer mobilen Herz-Lungen-Maschine funktionsfähig montiert werden, das heißt es ist nicht erforderlich, erst bei Inbetriebnahme des Systems Montageschritte oder Anpassungsmaßnahmen vorzunehmen.

Da der Strömungskanal zumindest abschnittsweise einen im Wesentlichen rechteckigen oder quadratischen Querschnitt aufweist, sind etwaige Totzonen innerhalb des Strömungsquerschnitts ausgeschlossen und eine turbulente Strömung innerhalb des Querschnitts wird vermieden.

Vorteilhafte Ausführungsformen der Erfindung sind in der Beschreibung, der Zeichnung sowie den Unteransprüchen beschrieben.

Nach einer ersten vorteilhaften Ausführungsform kann im Querschnitt gesehen zu zwei Seiten des Strömungskanals ein Ultraschallsensorelement, zum Beispiel ein Ultraschallsender und ein Ultraschallempfänger in Form einer Piezokeramik angeordnet sein. Hierdurch ist der Strömungskanal auf an sich bekannte Weise gut detektierbar, wobei jedoch durch die Integration der Ultraschallsensorelemente und des Strömungskanals in ein einziges Bauteil keine Koppelmedien vorgesehen oder wiederholt zugeführt werden müssen. Ein Austausch von aus dem Stand der Technik bekannten Gelpads kann wegfallen und der Luftblasensensor kann an beliebigen Orten verbaut werden, ohne dass eine gute Zugänglichkeit für ein Einlegen eines Schlauches erforderlich wäre.

Nach einer weiteren vorteilhaften Ausführungsform kann der Strömungskanal zumindest abschnittsweise zwei gegenüberliegende Wandabschnitte aufweisen, die im Wesentlichen parallel zueinander verlaufen. Auf diese Weise lässt sich der gesamte Strömungsquerschnitt besonders gut auf Luftblasen detektieren, da der Ultraschall von den - üblicherweise quaderförmigen - Piezokeramiken gut in das Innere des Strömungskanals eingekoppelt werden kann.

Nach einer weiteren vorteilhaften Ausführungsform können die Anschlussstücke so ausgebildet sein, dass auf diese Schläuche mit unterschiedlichen Innendurchmessern aufschiebbar sind. Auf diese Weise lässt sich der Luftblasensensor für verschiedene Schlauchdurchmesser universell einsetzen.

Nach einer weiteren vorteilhaften Ausführungsform kann an der Halterung ein elektrischer Steckverbinder für den Ultraschallsensor angeordnet sein, sodass dieser auf besonders einfache Weise an ein Gerät angekoppelt werden kann. Weiterhin können an der Halterung elektrische Bauteile zur Ansteuerung des Ultraschallsensors angeordnet sein. Auf diese Weise lassen sich Abstimmglieder und dergleichen, welche an die Sensorelemente angepasst sein müssen, bereits bei der Herstellung abstimmen, sodass der Luftblasensensor anschließend lediglich in ein zugehöriges Gerät eingesetzt werden muss, ohne dass es weiterer Einstellungen oder Abstimmmaßnahmen bedarf.

Nach einer weiteren vorteilhaften Ausführungsform kann für den Luftblasensensor eine Steckaufnahme vorgesehen sein, die beispielsweise an dem Gerät befestigt ist, mit welchem der Luftblasensensor verwendet werden soll. Eine solche Steckaufnahme kann insbesondere eine Halteklammer für den Luftblasensensor aufweisen, sodass bei der Vormontage der Luftblasensensor lediglich in die Halteklammer eingesetzt werden muss. Nach einem Einsatz des Luftblasensensors kann dieser auf äußerst einfache Weise aus der Steckaufnahme entfernt und beispielsweise entsorgt werden.

Nach einer vorteilhaften Ausführungsform kann in der Steckaufnahme eine Aussparung vorgesehen sein, in der bei eingestecktem Luftblasensensor ein Steckverbinder des Luftblasensensors geschützt untergebracht ist. Auf diese Weise erfolgt bei einem Einstecken des Luftblasensensors in die Steckaufnahme gleichzeitig eine elektrische Kontaktierung, wobei der elektrische Steckverbinder bzw. die elektrischen Bauteile des Luftblasensensors in der Aussparung der Steckaufnahme geschützt untergebracht sind.

Weiterhin kann nach einer vorteilhaften Ausführungsform in der Steckaufnahme eine Auswerteelektronik für den Luftblasensensor vorgesehen sein, sodass ohne lange elektrische Leitungswege im Bereich der Steckaufnahme eine Auswertung der detektierten Signale erfolgen kann.

Nach einer vorteilhaften Ausführungsform weist der Luftblasensensor eine Platine mit elektrischen oder elektronischen Bauteilen auf, welche den Strömungskanal zu zwei Seiten umgreift. Hierdurch lassen sich die Sensorelemente so auf der Platine anordnen, dass diese nach Aufschieben der Platine auf die Halterung zu beiden Seiten des Strömungskanals angeordnet sind.

Es kann vorteilhaft sein, wenn die Halterung einstückig und insbesondere aus einem für Ultraschall durchlässigen Material, beispielsweise einem Kunststoff, ausgebildet ist, da hierdurch der Luftblasensensor besonders wirtschaftlich hergestellt werden kann. Weiterhin kann der Luftblasensensor als Einwegteil ausgebildet sein, das heißt der Luftblasensensor, die Halterung und sämtliche an dieser angebrachten Teile können nach einem einmaligen Gebrauch entsorgt werden.

Nach einer weiteren vorteilhaften Ausführungsform der Erfindung verläuft der Strömungskanal in Durchströmungsrichtung geradlinig. Hierdurch wird eine turbulente Strömung, insbesondere im Bereich des Ultraschallsensors, verhindert.

Nachfolgend wird die vorliegende Erfindung rein beispielhaft anhand einer vorteilhaften Ausführungsform und unter Bezugnahme auf die beigefügten Zeichnungen beschrieben. Es zeigen:
- Fig. 1: eine Seitenansicht eines Luftblasensensors;
- Fig. 2: ein Schnitt durch den Luftblasensensor von Fig. 1 entlang der Linie A-A;
- Fig. 3: eine weitere Ausführungsform eines Luftblasensensors, der in eine Steckaufnahme eingesetzt ist;
- Fig. 4: den Luftblasensensor von Fig. 3 vor dem Einsetzen die Steckaufnahme; und
- Fig. 5: eine perspektivische Ansicht des Luftblasensensors der Fig. 3 und Fig. 4.

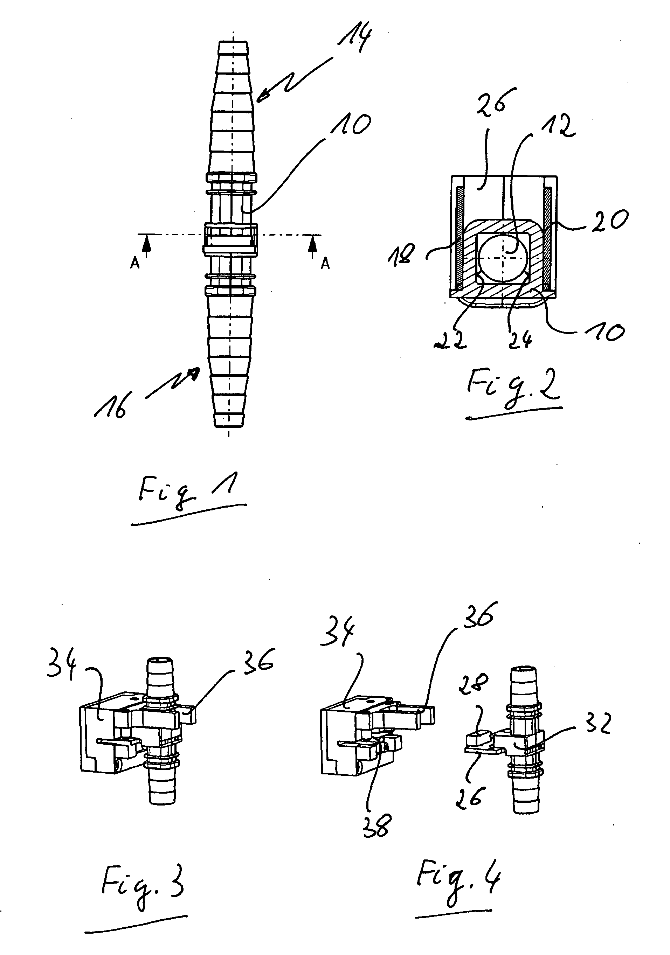

Der in Fig. 1 dargestellte Luftblasensensor weist eine aus Kunststoffmaterial einstückig hergestellte Halterung 10 auf, in der ein in Strömungsrichtung geradlinig verlaufender Strömungskanal 12 (vgl. Fig. 2) vorgesehen ist, der sich von einem Ende der Halterung 10 zu deren anderen Ende erstreckt. Die Halterung 10 weist die äußere Kontur eines Schlauchverbinders auf und besitzt ein oberes Anschlussstück 14 und ein unteres Anschlussstück 16 für jeweils einen Schlauch. Bei dem dargestellten Ausführungsbeispiel sind die beiden Anschlussstücke sich konisch verjüngend und gerastert ausgebildet, sodass Schläuche mit verschiedenem Innendurchmesser auf die Anschlussstücke aufgeschoben werden können. Die Anschlussstücke 14 und 16 sind in einzelne konische Abschnitte mit unterschiedlichen Außendurchmessern unterteilt, was ein Aufschieben der Schläuche mit verschiedenen Innendurchmessern erleichtert.

Fig. 2 zeigt einen Schnitt durch den Luftblasensensor von Fig. 1 entlang der Linie A-A, wobei erkennbar ist, dass im Querschnitt gesehen zu zwei Seiten des Strömungskanals 12 außen an der Halterung 10 jeweils ein Ultraschallsensorelement in Form einer Piezokeramik 18 und 20 angeordnet ist. Die Piezokeramiken 18 und 20 besitzen einen quaderförmigen plättchenförmigen Aufbau und sind an die Halterung angeklebt, die im Bereich der Piezokeramiken 18 und 20 zwei gegenüberliegende Außenwandabschnitte aufweist, die parallel zueinander verlaufen und an denen die Piezokeramiken befestigt sind. Im Bereich der Piezokeramiken 18 und 20, das heißt im Bereich der Messstrecke, ist der Querschnitt des Strömungskanals 12 quadratisch bzw. annähernd quadratisch ausgebildet, wodurch eine optimale Einkopplung der Ultraschallwellen in den Bereich der Messstrecke erfolgen kann. Wie Fig. 2 zeigt, verlaufen hierzu die Ultraschallsensorelemente 18, 20 und die sich gegenüberliegenden Innenwandabschnitte 22 und 24 des Strömungskanals 12 im Bereich der Messstrecke parallel zueinander. Außerhalb der Messstrecke geht der Querschnitt des Strömungskanals dann in einen runden Querschnitt über.

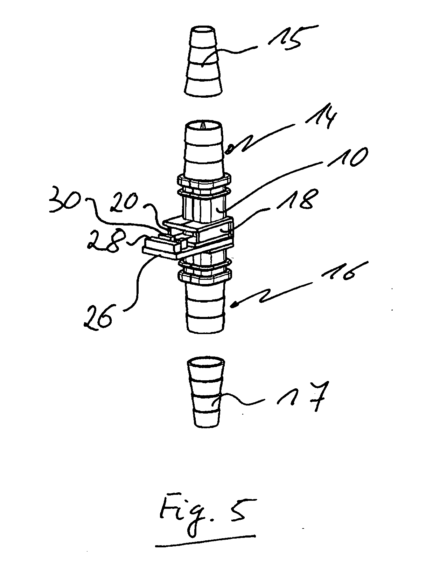

Fig. 5 zeigt den Luftblasensensor von Fig.1 und 2 in einer perspektivischen Ansicht, wobei lediglich im Bereich der-Anschlussstücke 14 und 16 äußere Endabschnitte 15 und 17 entfernt sind. Hierzu können beispielsweise an den Endabschnitten 15 und 17 Sollbruchstellen vorgesehen sein, die ein leichtes Entfernen ermöglichen.

Fig. 5 verdeutlicht, dass auf dem Luftblasensensor unterhalb der Ultraschallsensorelemente 18 und 20 eine Schaltplatine 26 angeordnet ist, deren Hauptfläche sich senkrecht zu der Strömungsrichtung erstreckt. Die Schaltplatine 26 umgreift den Strömungskanal bzw. die Messstrecke zu zwei Seiten, sodass die Ultraschallsensorelemente 18 und 20 auch auf der Schaltplatine 26 befestigt werden können. Weiterhin befindet sich am vorderen Ende der Schaltplatine 26 ein Steckverbinder 28, der eine elektrische Ankopplung des Ultraschallsensors ermöglicht. Auf der Schaltplatine 26 sind weiterhin elektrische Bauteile wie Abstimmglieder 30 vorgesehen, mit denen eine Anpassung an die verwendeten Ultraschallsensorelemente erfolgen kann.

Fig. 5 verdeutlicht, dass der Luftblasensensor 10, die Schaltplatine 26 sowie die darauf angebrachten Bauteile zu einer Einheit miteinander verbunden sind, die als solche gehandhabt werden kann, das heißt diese Einheit kann nach ihrer Herstellung im Werk vormontiert und mit entsprechenden Schläuchen verbunden werden. Nach einem Einsatz kann die Einheit von den Schläuchen abgesteckt und entsorgt werden.

Die Fig. 3 und 4 zeigen den Luftblasensensor der Fig. 5, wobei lediglich im Bereich der Sensorelemente 18 und 20 noch eine Verkleidung 32 vorgesehen ist. Die Fig. 3 und 4 zeigen weiterhin eine Steckaufnahme 34 für den Luftblasensensor, die eine Halteklammer 36 aufweist, in welche der Luftblasensensor eingesteckt werden kann. Weiterhin ist in der Steckaufnahme 34 eine Aussparung 38 vorgesehen, in der bei eingestecktem Luftblasensensor der Steckverbinder 28 sowie die Schaltplatine 26 geschützt untergebracht sind. Innerhalb der Steckaufnahme 34 befindet sich auch ein komplementäres Steckerteil, sodass nach Einsetzen des Luftblasensensors in die Steckaufnahme nicht nur eine mechanische Befestigung sondern auch gleich eine elektrische Kontaktierung erfolgt ist. In der Steckaufnahme 34 ist weiterhin eine (nicht dargestellte) Auswerteelektronik für den Luftblasensensor vorgesehen.

Der erfindungsgemäße Luftblasensensor kann direkt in ein Schlauchsystem integriert werden, ohne dass über ein Koppelmedium eine Ankopplung an das Schlauchsystem erfolgen muss. Es entfällt somit die fehleranfällige Einlage eines Schlauches und das Aufbringen eines Koppelmediums. Der Luftblasensensor ist direkt nach der Montage einsatzbereit und der Luftblasensensor kann auch unzugänglichen Stellen platziert werden. Der Sensor kann nach Verwendung und einmaligem Gebrauch aus der Steckaufnahme entnommen und mit dem übrigen Schlauchmaterial entsorgt werden. Die Auswerteelektronik kann allerdings in der Steckaufnahme verbleiben und für die folgende Verwendung wieder genutzt werden. Die Steckaufnahme kann an dem Gehäuse eines gewünschten Gerätes, beispielsweise einer mobilen Herz-Lungen-Maschine befestigt sein.

## Patentansprüche

1. Luftblasensensor mit einer Halterung (10), an der zumindest ein Ultraschallsensor angeordnet ist, um Luft- und/oder Gasblasen in einer strömenden Flüssigkeit zu detektieren, wobei
in die Halterung (10) ein geschlossener Strömungskanal (12) integriert ist, der zwei Anschlussstücke (14, 16) für jeweils einen Schlauch aufweist, **dadurch gekennzeichnet, dass** der Strömungskanal (12) zumindest abschnittsweise einen im wesentlichen rechteckigen oder quadratischen Querschnitt aufweist.

2. Luftblasensensor nach Anspruch 1,
**dadurch gekennzeichnet, dass**
im Querschnitt gesehen zu zwei Seiten des Strömungskanals (12) ein Ultraschallsensorelement (18, 20) angeordnet ist.

3. Luftblasensensor nach Anspruch 1 oder 2,
dadurchgekennzeichnet,dass
der Strömungskanal (12) zumindest abschnittsweise zwei gegenüberliegende Wandabschnitte (22, 24) aufweist, die im Wesentlichen parallel zueinander verlaufen.

4. Luftblasensensor nach zumindest einem der vorstehenden Ansprüche,
**dadurch gekennzeichnet, dass**
die Anschlussstücke (14, 16) so ausgebildet sind, dass auf diese Schläuche mit unterschiedlichen Innendurchmessern aufschiebbar sind.

5. Luftblasensensor nach zumindest einem der vorstehenden Ansprüche,
**dadurch gekennzeichnet, dass**
an der Halterung (10) ein elektrischer Steckverbinder für den Ultraschallsensor angeordnet ist.

6. Luftblasensensor nach zumindest einem der vorstehenden Ansprüche,
**dadurch gekennzeichnet, dass**
an der Halterung (10) elektrische Bauteile (30) zur Ansteuerung des Ultraschallsensors (18, 20) angeordnet sind.

7. Luftblasensensor nach zumindest einem der vorstehenden Ansprüche,
**dadurch gekennzeichnet, dass**
dieser in eine Steckaufnahme (34) eingesetzt ist, die insbesondere eine Halteklammer (36) für den Luftblasensensor aufweist.

8. Luftblasensensor nach Anspruch 7,
**dadurch gekennzeichnet, dass**
in der Steckaufnahme (34) eine Aussparung (38) vorgesehen ist, in der ein Steckverbinder (28) des Luftblasensensors geschützt untergebracht ist.

9. Luftblasensensor nach Anspruch 7 oder 8,
**dadurch gekennzeichnet, dass**
in der Steckaufnahme (34) eine Auswerteelektronik für den Luftblasensensor vorgesehen ist.

10. Luftblasensensor nach zumindest einem der vorstehenden Ansprüche,
**dadurch gekennzeichnet, dass**
dieser eine Platine (26) aufweist, die den Strömungskanal (12) zu zwei Seiten umgreift.

11. Luftblasensensor nach zumindest einem der vorstehenden Ansprüche,
**dadurch gekennzeichnet, dass**
dieser die äußere Kontur eines Schlauchverbinders aufweist.

12. Luftblasensensor nach zumindest einem der vorstehenden Ansprüche,
**dadurch gekennzeichnet, dass**
die Halterung (10) einstückig und insbesondere aus einem für Ultraschall durchlässigen Material ausgebildet ist.

13. Luftblasensensor nach zumindest einem der vorstehenden Ansprüche,
**dadurch gekennzeichnet, dass**
der Strömungskanal (12) in Durchströmungsrichtung geradlinig verläuft.

## Claims

1. An air bubble sensor having a holder (10) at which at least one ultrasonic sensor is arranged to detect air bubbles and/or gas bubbles in a flowing liquid, wherein
a closed flow passage (12) which has two connection pieces (14, 16) each for a respective tube is integrated into the holder (10),
**characterized in that**
the flow passage (12) has, at least sectionally, a substantially rectangular or square cross-section.

2. An air bubble sensor in accordance with claim 1,
**characterized in that**
an ultrasonic sensor element (18, 20) is arranged at two sides of the flow passage (12) viewed in cross-section.

3. An air bubble sensor in accordance with claim 1 or claim 2,
**characterized in that**
the flow passage (12) has, at least sectionally, two oppositely disposed wall sections (22, 24) which extend substantially parallel to one another.

4. An air bubble sensor in accordance with at least one of the preceding claims,
**characterized in that**
the connection pieces (14, 16) are configured so that tubes having different inner diameters can be pushed onto them.

5. An air bubble sensor in accordance with at least one of the preceding claims,
**characterized in that**
an electric plug connector for the ultrasonic sensor is arranged at the holder (10).

6. An air bubble sensor in accordance with at least one of the preceding claims,
**characterized in that**
electric components (30) for controlling the ultrasonic sensor (18, 20) are arranged at the holder (10).

7. An air bubble sensor in accordance with at least one of the preceding claims,
**characterized in that**
it is inserted into a plug receiver (34) which in particular has a holding clamp (36) for the air bubble sensor.

8. An air bubble sensor in accordance with claim 7,
**characterized in that**
a cut-out (38) is provided in the plug receiver (34) and a plug connector (28) of the air bubble sensor is arranged therein in a protected manner.

9. An air bubble sensor in accordance with claim 7 or claim 8,
**characterized in that**
evaluation electronics for the air bubble sensor are provided in the plug receiver (34).

10. An air bubble sensor in accordance with at least one of the preceding claims,
**characterized in that**
it has a circuit board (26) which engages around the flow passage (12) at two sides.

11. An air bubble sensor in accordance with at least one of the preceding claims,
**characterized in that**
it has the outer contour of a tube connector.

12. An air bubble sensor in accordance with at least one of the preceding claims,
**characterized in that**
the holder (10) is configured in one piece and in particular from a material permeable for ultrasound.

13. An air bubble sensor in accordance with at least one of the preceding claims,
**characterized in that**
the flow passage (12) extends in a straight line in the throughflow direction.

## Revendications

1. Détecteur de bulles comprenant une monture (10) sur laquelle est agencé au moins un détecteur à ultrasons, afin de détecter des bulles d'air et/ou de gaz dans un liquide en écoulement, dans lequel un canal d'écoulement fermé (12) est intégré dans la monture (10), lequel comporte deux pièces de raccordement (14, 16) pour un tuyau flexible respectif,
**caractérisé en ce que**
le canal de l'écoulement (12) présente au moins localement une section sensiblement rectangulaire ou carrée.

2. Détecteur de bulles selon la revendication 1,
**caractérisé en ce qu'**un élément détecteur à ultrasons (18, 20) est agencé, vu en section transversale, sur les deux côtés du canal d'écoulement (12).

3. Détecteur de bulles selon la revendication 1 ou 2,
**caractérisé en ce que** le canal d'écoulement (12) comporte au moins localement deux tronçons de paroi opposés (22, 24) qui s'étendent sensiblement parallèlement l'un à l'autre.

4. Détecteur de bulles selon l'une au moins des revendications précédentes,
**caractérisé en ce que** les pièces de raccordement (14, 16) sont réalisées de telle façon qu'il est possible d'enfiler sur celles-ci des tuyaux flexibles avec des diamètres intérieurs différents.

5. Détecteur de bulles selon l'une au moins des revendications précédentes,
**caractérisé en ce qu'**un connecteur électrique enfichable pour le détecteur à ultrasons est agencé sur la monture (10).

6. Détecteur de bulles selon l'une au moins des revendications précédentes,
**caractérisé en ce que** des composants électriques (30) pour le pilotage du détecteur à ultrasons (18, 20) sont agencés sur la monture (10).

7. Détecteur de bulles selon l'une au moins des revendications précédentes,
**caractérisé en ce que** celui-ci est mis en place dans un récepteur à enfichage (34) qui comporte en particulier une pince de maintien (36) pour le détecteur de bulles.

8. Détecteur de bulles selon la revendication 7,
**caractérisé en ce qu'**un évidement (38) est prévu dans le récepteur à enfichage (34), dans lequel un connecteur enfichable (28) du détecteur de bulles est logé de manière protégée.

9. Détecteur de bulles selon la revendication 7 ou 8,
**caractérisé en ce qu'**une unité électronique d'évaluation pour le détecteur de bulles est prévue dans le récepteur à enfichage (34).

10. Détecteur de bulles selon l'une au moins des revendications précédentes,
**caractérisé en ce que** celui-ci comprend une platine (26) qui entoure le canal d'écoulement (12) sur deux côtés.

11. Détecteur de bulles selon l'une au moins des revendications précédentes,
**caractérisé en ce que** celui-ci présente le contour extérieur d'un connecteur à tuyau flexible.

12. Détecteur de bulles selon l'une au moins des revendications précédentes,
**caractérisé en ce que** la monture (10) est réalisée d'une seule pièce, et en particulier en un matériau perméable aux ultrasons.

13. Détecteur de bulles selon l'une au moins des revendications précédentes,
**caractérisé en ce que** le canal d'écoulement (12) s'étend en ligne droite dans la direction de l'écoulement traversant.
